# EUROPEAN PATENT APPLICATION

(11) **EP 4 223 227 A1**
(43) Date of publication of application: **09.08.2023**
(21) Application number: 22290004.5
(22) Date of filing: 03.02.2022
(51) Int. Cl.: A61B 8/08, G06T 7/10

(54) **A METHOD AND SYSTEM FOR PERFORMING FETAL WEIGHT ESTIMATIONS**

(71) Applicant: Koninklijke Philips N.V., 5656 AG Eindhoven (NL)
(72) Inventor: Raynaud, Caroline Denise Francoise, 5656 AE Eindhoven (NL); Olivier, Antoine, 5656 AE Eindhoven (NL); Ciofolo-Vei, Cybèle, 5656 AE Eindhoven (NL); Rouet, Laurence, 5656 AE Eindhoven (NL)
(74) Representative: Philips Intellectual Property & Standards

(57) **Abstract**

A fetal weight estimation is made by segmenting the fetal spine from a 2D sagittal image of the spine and segmenting the spine from a 3D image of the torso. The two segmentations are registered, and the torso is segmented from the 3D image. If the 3D image is missing a portion of the torso, the missing portion of the torso is estimated based on the registered spine segmentations and the torso segmentation. A complete torso volume can then be estimated by extrapolating from the part of the torso present in the 3D image, and an accurate fetus weight estimation may be made.

## Description

### FIELD OF THE INVENTION

This invention relates to the field of ultrasound imaging, and in particular to the field of fetal ultrasound imaging.

### BACKGROUND OF THE INVENTION

In current clinical practice, fetal weight is typically estimated using a combination of 2D measurements, extracted from 2D ultrasound images. The measurements are performed on 2D standard clinical planes. Fetal weight estimation is very important in the management of the pregnancy but is often under or over estimated.

Where the fetus is too small or too large, errors in fetal weight estimation during pregnancy may lead to inadequate care given to the infant during pregnancy or after birth. Depending on the actual weight of the fetus, the balance between relative coefficients used in the current models will affect the precision of the estimated fetal weight. For example, in the case of fetal weights over 3500g, formulae that depend more on the abdominal circumference and femur length in the calculation provide more accurate predictions of birth weight.

Current equations used for fetal weight estimation are based on statistical regressions and estimations based on population analysis. The number of different equations is very high, which leads to various possible estimates according to the selected model and introduces uncertainty in which model to apply.

In order to improve accuracy, it is known to use fractional limb volume as an additional parameter in fetal weight equations, such as thigh and arm fractional volumes. However, there remain inaccuracies in the fetal weight estimation.

It is also known to use 3D images to perform fetal weight estimation. WO 2020/011569 discloses the use of multiple 3D ultrasound measurements in order to make fetal weight estimation more robust. A head image, abdominal image and femur image are each segmented and the fetal weight estimation is based on the resulting segmentations. However, these measurements may be difficult to obtain, as the fetus does not necessarily fit in one 3D acquisition. This is especially the case for the torso of the fetus after 20 weeks. It is then usually too large to fit in one single imaging volume.

A partial torso covering will lead to an under estimation of the torso volume and thus of the torso weight. Thus, it is necessary to obtain an estimation of the fraction of the torso that is missing in the 3D acquisition.

There is therefore a need for a practical method and system that is able to estimate accurately a torso volume from a partial 3D torso acquisition, to enable more accurate fetal weight estimation from a 3D fetus image.

### SUMMARY OF THE INVENTION

The invention is defined by the claims.

According to examples in accordance with an aspect of the invention, there is provided a method for performing fetal weight estimation, the method comprising:
receiving at least one 2D image of a sagittal view of the spine of the fetus;
receiving at least one 3D image of the torso of the fetus;
segmenting the spine from the at least one 2D image to create a first segmentation and segmenting the spine from the at least one 3D image to create a second segmentation;
registering the first and second segmentations;
segmenting the torso from the at least one 3D image to create a torso segmentation;
estimating a missing portion of the torso within the at least one 3D image based on the registered first and second segmentations and the torso segmentation;
estimating a complete torso volume using the at least one 3D image, based on the estimation of the missing portion; and
estimating a fetus weight based on the estimated complete torso volume.

The 2D and 3D images are preferably ultrasound images. The 3D image for example covers only a portion of the torso and hence only a portion of the spine, whereas the the 2D image cover more of the spine, and preferably the entire spine of the fetus.

The method estimates a missing portion of the torso from the 3D image. The 3D shape of the missing portion of the torso may be estimated, or there may simply be an estimation of the missing volume as a fraction i.e. the percentage volume of the torso that is missing from the 3D image.

The full torso 3D volume may be considered to be extrapolated from the portion of the volume that is present in the 3D image. However, instead of extrapolating based only on the information present in the 3D image itself, a spine segmentation from the 2D image, which covers more and preferably all of the spine, enables the extrapolation to be more accurate. In particular, the 3D volume that is present is extrapolated with knowledge of more (and preferably all) of the spine shape. For example, a torso shape model may be fitted not only to the 3D image data that is present, but also to the additional spine portions obtained from the 2D sagittal image. Thus, the torso shape model may be applied more accurately and give more accurate volume and weight estimations than when applied to the 3D image alone.

The method for example involves creating the first and second segmentations using a Deep Learning model. The first and second segmentations may for example be obtained using landmark detection. Such landmark detection may use a pose estimation network or an object detection network.

Registering the first and second segmentations for example comprises matching spinal features, in particular vertebrae, between the first and second segmentations. As well as matching spinal features, the registering may also comprise matching additional non-spinal landmarks, such as the heart, stomach etc.

Creating the torso segmentation may also be implemented using a Deep Learning model.

The method may comprise estimating a missing portion of the torso by fitting an ellipsoidal torso shape model to the torso segmentation.

The method for example comprises displaying the torso segmentation and the missing portion to a user. This allows the user to assess if the extrapolation looks correct. The method may then further comprise receiving user input based on the displayed torso segmentation and missing portion to instruct adjustment of the shape of the missing portion. Thus, the user can make adjustments to the extrapolation, and the volume and weight estimations will then be adjusted accordingly.

The fetal weight estimation is for example based on a global homogeneous tissue density.

The invention also provides a computer program comprising computer program code means which is adapted, when said computer program is run on a computer, to implement the method defined above.

The invention also provides an ultrasound imaging system comprising:
an ultrasound probe adapted to acquire two dimensional ultrasound images of an imaging region;
an ultrasound probe adapted to acquire three dimensional ultrasound images of the imaging region;
a display; and
a processor, wherein the processor is adapted to:
   receive at least one 2D image of a sagittal view of the spine of the fetus;
   receive at least one 3D image of the torso of the fetus;
   segment the spine from the at least one 2D image to create a first segmentation and segment the spine from the at least one 3D image to create a second segmentation;
   register the first and second segmentations;
   segment the torso from the at least one 3D image to create a torso segmentation;
   estimate a missing portion of the torso within the at least one 3D image based on the registered first and second segmentations and the torso segmentation;
   estimate a complete torso volume using the at least one 3D image, based on the estimation of the missing portion; and
   estimate a fetus weight based on the estimated complete torso volume.

The processor is for example adapted to register the first and second segmentations by matching vertebrae between the first and second segmentations. The missing portion of the torso is for example estimated by fitting an ellipsoidal torso shape model to the torso segmentation.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an ultrasound diagnostic imaging system to explain the general operation;
Fig. 2 shows a 2D ultrasound image which is a sagittal view of a fetus;
Fig. 3 shows a 3D ultrasound image of a fetus;
Fig. 4 shows a method of the invention;
Fig. 5 shows how model fitting may be applied to a 3D image with partial torso coverage; and
Fig. 6 shows how a use may adapt the image of Figure 5 to adapt the way missing torso coverage is corrected.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for estimating the weight of a fetus. The fetal weight estimation is made by segmenting the fetal spine from a 2D sagittal image of the spine and segmenting the fetal spine from a 3D image of the torso. The two segmentations are registered, and the torso is segmented from the 3D image. If the 3D image is missing a portion of the torso, the missing portion of the torso is estimated based on the registered spine segmentations and the torso segmentation. A complete torso volume can then be estimated by extrapolating from the part of the torso present in the 3D image, and an accurate fetus weight estimation may be made. Thus, the use of a 2D sagittal image covering a larger volume of the torso than a 3D image enables increased accuracy in deriving a fetal volume and weight estimation from the 3D image.

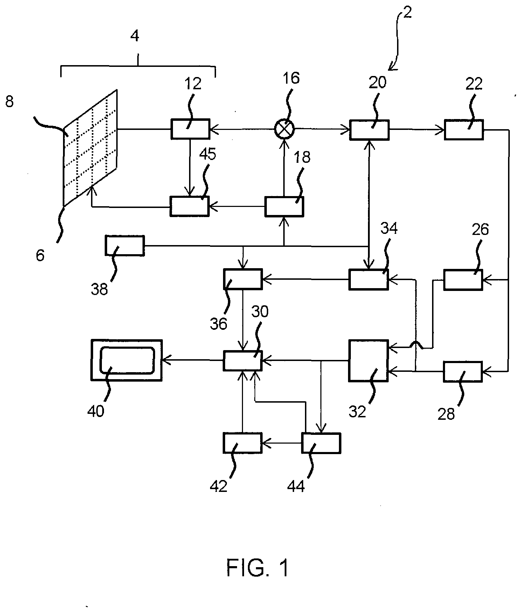

The general operation of an exemplary ultrasound system will first be described, with reference to Fig. 1. The system produces ultrasound images, and these are then processed in the manner explained further below in order to implement the method of the invention.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest.

This invention in particular has both 2D imaging functionality and 3D imaging functionality.

Optionally, the transducer array 6 is coupled to a microbeamformer 12 which controls reception of signals by the transducer elements. Microbeamformers are capable of at least partial beamforming of the signals received by sub-arrays.

The system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

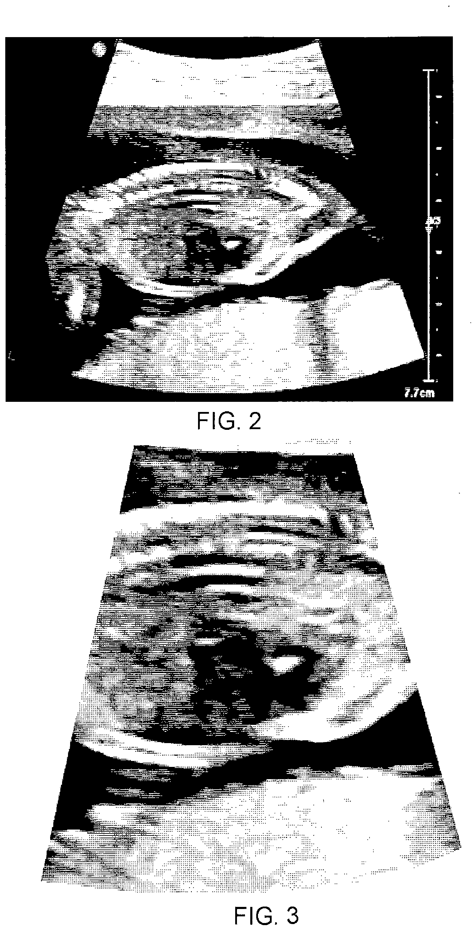

Fig. 2 shows a 2D acquisition of a sagittal view of the spine. This is part of the clinical screening routine in Fetal Ultrasound. The 2D ultrasound field of view is less constrained than in 3D, which enables the user to perform a 2D acquisition where the whole spine of the fetus is visible throughout most of the pregnancy.

However, as the fetus gets bigger, it is less likely that a whole torso would fit in a 3D acquisition, leading to a partial torso volumetric imaging as shown in Fig. 3.

The invention is based on the use of the information from a 2D sagittal view of the spine to infer which portion of the torso is missing in a 3D acquisition.

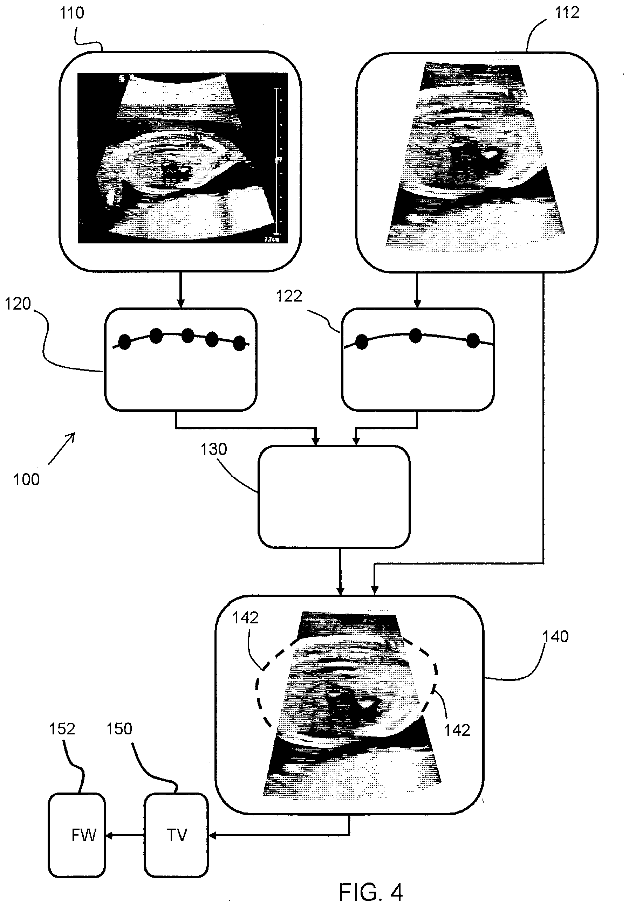

Fig. 4 shows the steps of a method 100 for performing fetal weight estimation.

In step 110 a 2D ultrasound acquisition of a sagittal view of the spine is implemented. The user is for example responsible for the acquisition of an appropriate 2D sagittal view in known manner.

In step 112, a 3D ultrasound acquisition of the torso is implemented. The obtained 3D image may cover the torso only partially, as explained above.

The 2D and 3D images may be obtained from separate probes used in sequence, or from a 3D probe with 2D acquisition capability.

In step 120, image processing is performed to segment the spine from the 2D sagittal view image.

In step 122, image processing is performed to segment the spine from the 3D image. This is a segmentation to obtain the spine shape in 3D.

The image processing to perform spine segmentation can be performed using Deep learning methods, such as a U-Net model, or any kind of segmentation model. Landmarks can be detected along the spine, for example detecting predefined precise vertebrae. Landmark detection can be implemented using pose estimation networks in both 3D and 2D (e.g., HRnet), or object detection networks in 2D (e.g., YOLO, FastRCNN). The identification of landmarks assists in the registration of the spine between the two images, in step 130.

By way of example, the spine may be automatically detected in a 3D ultrasound abdominal image by combining a morphological filter which detects elongated bright structures and a deep learning (DL) based vertebrae detector, in order to take advantage of the strengths of both methods.

A morphological filter may be used for each voxel in the abdominal image volume in a given spherical neighborhood, to compare the intensity of the voxels along a direction with the intensity of the other voxels. The filter responses are computed for various neighborhood radii and orientations and combined to obtain a global response. The global responses of neighboring voxels are aggregated to define connected components which correspond to the best filter responses.

Although some of the responses are accurately positioned on the spine using this approach, others may also be present which are outliers, that may for example be located on ribs or other elongated structures such as long bones.

The deep learning-based vertebrae detector is a 2D fully convolutional network whose input is made of 2D slices, extracted orthogonally to an image-based z-axis. The volume slicing produces a large amount of data with similar features, which is appropriate for deep learning methods. The network output is a down-sampled probability map, with values closer to 1 where the spine might be located. A 3D deep learning-based vertebrae detector is formed by stacking all the obtained 2D probability maps for one volume. This output heatmap is coarser than the morphological filter output, but more robustly located around the vertebrae.

By combining the deep-learning vertebrae detector and the morphological filter responses, the network output is refined and the filter responses that are outside the spine are rejected, so that a robust spine binary mask is finally obtained for the abdominal image.

This is one way to identify the spine location, but any other suitable image processing techniques may be employed for detecting the unique spine shape.

Registering the two segmentations of the spine in step 130 is performed based on the spine segmentations. Each vertebrae in both the 2D image and the 3D image are for example matched using the landmark (vertebrae) identification performed during the segmentation steps 120, 122.

The registration step can take place be between the 3D volume and the 2D image directly, e.g., taking the position of the vertebrae. Alternatively, a sagittal plane can be derived from the 3D image so that that registration then takes place between the spine segmentations in corresponding sagittal views.

Additional landmarks may also be used, such as the heart, the stomach or the umbilical vein. Localizing any of these landmarks on both the 2D and 3D views will help in the registering of the two image acquisitions. As mentioned above, this landmark detection task can be done using pose estimation or object detection networks.

In step 140, image processing is used to segment the torso from the 3D image. This can be performed using Deep learning methods, such as a 3D U-Net model. The torso segmentation is a 3D segmentation applied to the 3D image.

It is possible also to use a 2D segmentation of the torso, for instance to better refine the final volume, as it provides information on the torso shape after the registration step.

The registration between the spines in the two images, and the torso segmentation, enables an evaluation of which portion of the torso is missing from the volume captured in the 3D image, shown as regions 142 in Fig. 4.

Thus, the spine shape captured from the 2D image, and the registration between the partial spine portion in the 3D image and the spine in the 2D image, enable the full spine shape, and hence the full torso size and shape, to be extrapolated from the 3D image of a partial volume.

An accurate torso volume and/or weight estimation can then be performed using volume calculations from the extrapolated 3D image. A complete torso volume is estimated in step 150 using the at least one 3D image, based on the estimation of the missing portion of the torso, and a fetus weight is estimated in step 152 based on the estimated complete torso volume.

The fetal weight estimation may for example be performed using a global homogeneous tissue density. In other words, the densities of all the different tissues of the fetus may be averaged, thereby generating a unique density coefficient, and multiplied by the fetal volume discerned from the extrapolated image segmentation in order to perform a fetal weight estimation with minimal computational cost. The average density of a fetus may be defined based on relevant literature.

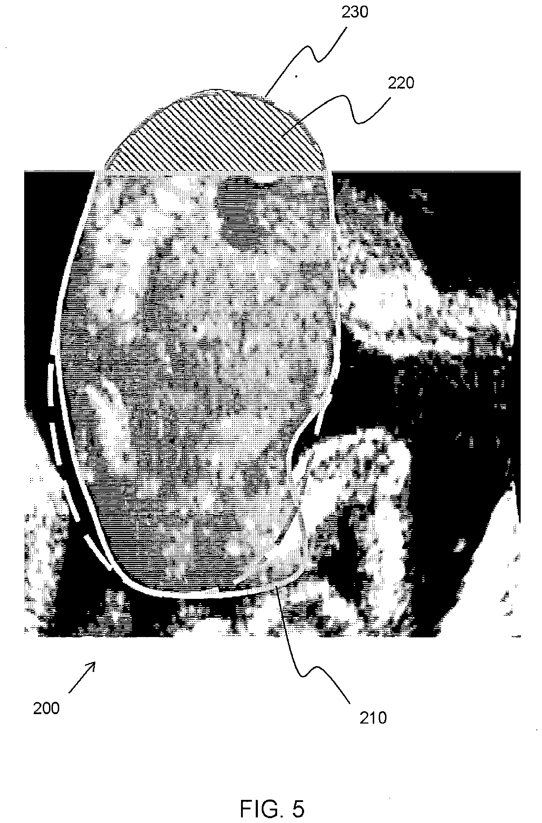

The extrapolation for example may comprise fitting an ellipsoidal torso shape model to the torso segmentation, as illustrated in Fig. 5.

Fig. 5 shows an abdominal image 200 comprising a partial view of the torso of the fetus.

Using a normal segmentation operation, the torso of the fetus is identified as indicated by the solid line outline 210. However, due to the partially incomplete view of the torso, the segmentation excludes the missing information 220, which would otherwise form the remaining portion of the torso of the fetus. This missing information would reduce the accuracy of the final fetal weight estimation.

The partial imaging of the torso may be compensated by way of a model torso image. The model torso image, in this case a model 230 with an ellipsoidal fitting, is fit to the segmented partial torso of the fetus as explained above, using both the data of the 3D image and the spine shape obtained from the 2D sagittal image, which thus extends into the missing region.

The model 230 may thus be applied more accurately to the segmented torso portion for example to obtain a ratio of the visible volumes and the total volume. Finally, the segmented torso is multiplied by the obtained ratio in order to compensate for the missing information.

The accuracy of the estimated fetal weight may be improved by identifying the volumes of the various tissues present within a segmented image, instead of assuming a homogeneous density.

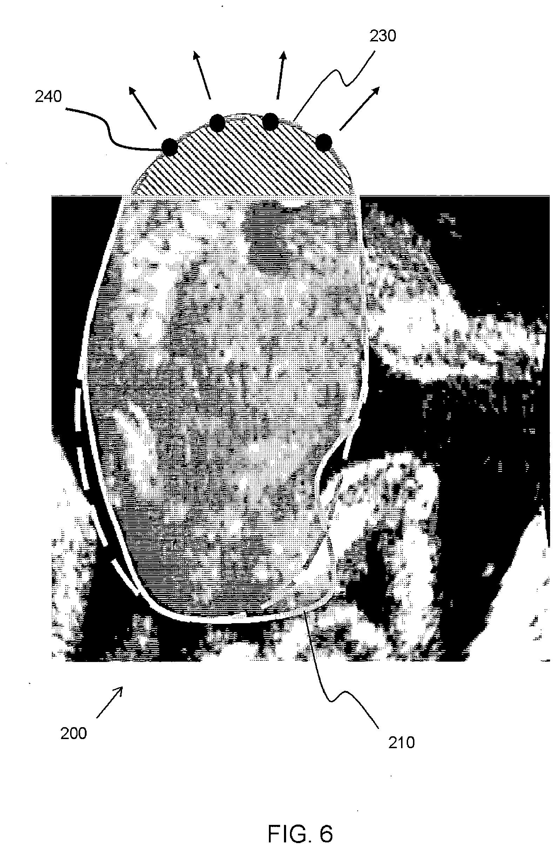

On a graphical user interface, control points 240 may be added to allow for manual correction by the user, as illustrated in Fig. 6. If a control point 240 is moved by the clinician, the border of the torso is updated accordingly.

In this case, only a first step of torso completion is performed automatically using AI or non-AI algorithms. The user is then presented with the results of this automatic torso extrapolation process and can decide to accept the results, or correct the results manually by moving come control points on the completed region.

Once a final torso segmentation is accepted, a volume and/or weight estimation can then be computed automatically.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for performing fetal weight estimation, the method comprising:
(110) receiving at least one 2D image of a sagittal view of the spine of the fetus;
(112) receiving at least one 3D image of the torso of the fetus;
(120) segmenting the spine from the at least one 2D image to create a first segmentation and (122) segmenting the spine from the at least one 3D image to create a second segmentation;
(130) registering the first and second segmentations;
(140) segmenting the torso from the at least one 3D image to create a torso segmentation; estimating a missing portion (142) of the torso within the at least one 3D image based on the registered first and second segmentations and the torso segmentation;
(150) estimating a complete torso volume using the at least one 3D image, based on the estimation of the missing portion; and
(152) estimating a fetus weight based on the estimated complete torso volume.

2. The method of claim 1, comprising creating the first and second segmentations using a Deep Learning model.

3. The method of any one of claims 1 to 2, comprising creating the first and second segmentations using landmark detection.

4. The method of claim 3, wherein the landmark detection uses a pose estimation network or an object detection network.

5. The method of one of claims 1 to 4, wherein registering the first and second segmentations comprises matching vertebrae between the first and second segmentations.

6. The method of any one of claims 1 to 5, wherein registering the first and second segmentations comprises matching additional non-spinal landmarks.

7. The method of any one of claims 1 to 6, comprising creating the torso segmentation using a Deep Learning model.

8. The method of any one of claims 1 to 7, comprising estimating a missing portion (142) of the torso by fitting an ellipsoidal torso shape model to the torso segmentation.

9. The method of any one of claims 1 to 8, wherein the method further comprises displaying the torso segmentation and the missing portion to a user.

10. The method of claim 9, wherein the method further comprises receiving user input based on the displayed torso segmentation and missing portion to instruct adjustment of the shape of the missing portion.

11. The method of any of claims 1 to 10, wherein the fetal weight estimation is based on a global homogeneous tissue density.

12. A computer program comprising computer program code means which is adapted, when said computer program is run on a computer, to implement the method of any of claims 1 to 11.

13. An ultrasound imaging system comprising:
an ultrasound probe adapted to acquire two dimensional ultrasound images of an imaging region;
an ultrasound probe adapted to acquire three dimensional ultrasound images of the imaging region;
a display; and
a processor, wherein the processor is adapted to:
(110) receive at least one 2D image of a sagittal view of the spine of the fetus;
(112) receive at least one 3D image of the torso of the fetus;
(120) segment the spine from the at least one 2D image to create a first segmentation and (122) segment the spine from the at least one 3D image to create a second segmentation;
(130) register the first and second segmentations;
(140) segment the torso from the at least one 3D image to create a torso segmentation;
estimate a missing portion (142) of the torso within the at least one 3D image based on the registered first and second segmentations and the torso segmentation;
(150) estimate a complete torso volume using the at least one 3D image, based on the estimation of the missing portion; and
(152) estimate a fetus weight based on the estimated complete torso volume.

14. The system of claim 13, wherein the processor is adapted to register the first and second segmentations by matching vertebrae between the first and second segmentations.

15. The system of claim 13 or 14, wherein the processor is adapted to estimate a missing portion (142) of the torso by fitting an ellipsoidal torso shape model to the torso segmentation.
